(19) Europäisches Patentamt
European Patent Office
Office européen des brevets

(11) Publication number: **0 264 114 A1**

(12) **EUROPEAN PATENT APPLICATION**

(21) Application number: 87114979.5

(22) Date of filing: 14.10.87

(51) Int. Cl.4: C07D 213/30, A61K 31/44

(30) Priority: 16.10.86 US 919723

(43) Date of publication of application: 20.04.88 Bulletin 88/16

(84) Designated Contracting States: AT BE CH DE ES FR GB GR IT LI NL SE

(71) Applicant: G.D. Searle & Co.
P.O. Box 5110
Chicago Illinois 60680(US)

(72) Inventor: Lai, Steve Mun Fook
179 River Valley Road
Singapore(SG)
Inventor: Manley, Paul William
Hermann Suter Strasse 9
CH-4053 Basel(CH)
Inventor: Porter, Roderick Alan
2 The Ferns Finings Road
Lane End High Wycombe(GB)

(74) Representative: Beil, Hans Chr., Dr. et al
Beil, Wolff und Beil, Rechtsanwälte
Adelonstrasse 58 Postfach 80 01 40
D-6230 Frankfurt am Main 80(DE)

(54) **Alpha-[(phenylmethoxy)methyl]pyridine-alkanol derivatives.**

(57) This invention relates to novel α-[(phenylmethoxy)methyl]-pyridinealkanol derivatives useful in the treatment of diseases or disorders mediated by platelet-activating factor. This invention further relates to pharmaceutical compositions of such α-[(phenylmethoxy)methyl]pyridinealkanol derivatives.

EP 0 264 114 A1

# α-[(PHENYLMETHOXY)METHYL]PYRIDINEALKANOL DERIVATIVES

## BACKGROUND OF THE INVENTION

Platelet-activating factor (PAF) has been associated with various biologic activities and pathways, thus making it an important mediator responsible for a variety of physiologic processes including, but not limited to, activation or coagulation of platelets, smooth muscle contraction, pathogenesis of immune complex deposition, inflammation, endotoxin shock, and respiratory, cardiovascular and intravascular alterations. These physiological processes are associated with a large group of diseases, such as, for example, cardiovascular disorders, asthma, lung edema, adult respiratory distress syndrome and inflammatory diseases.

European Patent Application Nos. 142,333 and 142,801 disclose a class of gylcerol derivatives and indene derivatives respectively, which are used as PAF-antagonists.

## SUMMARY OF THE INVENTION

The present invention relate to a novel class of compounds of the formula:

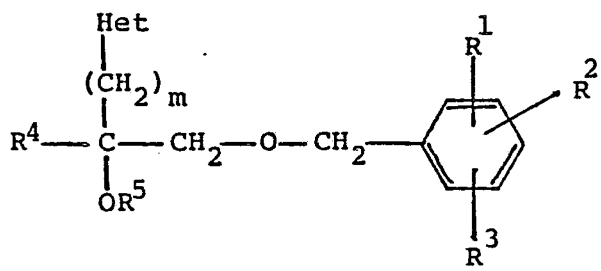

I

wherein $R^1$, $R^2$ and $R^3$ are independently hydrogen or $C_1$-$C_8$ alkoxy;
$R^4$ is hydrogen or $C_1$-$C_8$ alkyl;
$R^5$ is hydrogen, $-\overset{O}{\overset{\|}{C}}-R^6$, or $C_1$-$C_8$ alkyl,
wherein $R^6$ is $C_1$-$C_8$ alkyl;
m is an integer of from 1 to 4; and
Het is pyridyl;
or a pharmaceutically acceptable acid addition salt thereof.

The invention further relates to pharmaceutical compositions comprising a compound of formula (I). Such compounds and compositions have potent and specific PAF antogonistic activities and are thereby useful in the treatment of various diseases or disorders mediated by the PAF, for example, inflammation, cardiovascular disorder, asthma, lung edema, and adult respiratory distress syndrome.

## DETAILED DESCRIPTION

As used herein the term "$C_1$-$C_8$ alkyl" refers to straight chain or branched chain hydrocarbon groups having from one to eight carbon atoms. Illustrative of such alkyl groups include methyl, ethyl, propyl, isopropyl, butyl, isobutyl, sec-butyl, tert-butyl, pentyl, neopentyl, hexyl, isohexyl, heptyl, octyl and the like.

As used herein the term "$C_1$-$C_8$ alkoxy" refers to straight chain or branched chain alkoxy radicals having from one to eight carbons. Illustrative of such alkoxy groups are methoxy, ethoxy, propoxy, isopropoxy, butoxy, tert-butoxy, pentoxy, hexoxy, heptoxy, octoxy and the like.

The term "pharmaceutically acceptable acid addition salt" refers to a salt prepared by contacting a compound of formula (I) with an acid whose anion is generally considered suitable for human consumption. Examples of pharmacologically acceptable acid addition salts include the hydrochloride, hydrobromide, hydroiodide, sulfate, phosphate, acetate, propionate, lactate, maleate, malate, succinate, and tartrate salts.

The preferred compounds of the present invention include compounds of the formula

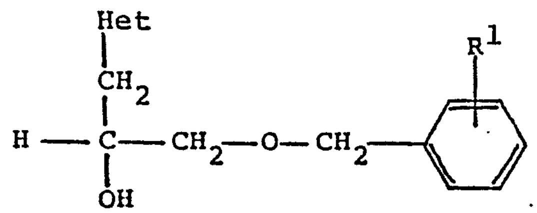

II

wherein $R^1$ is $C_1$-$C_8$ alkoxy and Het is pyridyl. A more preferred embodiment includes compounds of formula (II) wherein $R^1$ is methoxy and Het is pyridyl.

This invention also relates to a method of treatment for patients (or mammalian animals raised in the dairy, meat, or fur industries or as pets) suffering from disorders or diseases which can be attributed to PAF as previously described, and more specifically, a method of treatment involving the administration of compound (I) as the active ingredient.

Accordingly, compound (I) can be used among other things to reduce inflammation, to correct respiratory, cardiovascular, and intravascular alterations or disorders, and to regulate the activation or coagulation of platelets, the pathogenesis of immune complex deposition and smooth muscle contractions.

For the treatment of inflammation, cardiovascular disorder, asthma, or other diseases mediated by PAF, compound (I) may be administered orally, topically, parenterally, or by inhalation spray or rectally in dosage unit formulations containing conventional non-toxic pharmaceutically acceptable carriers, adjuvants and vehicles. The term parenteral as used herein includes subcutaneous, intravenous, intramuscular, or intrasternal injection or infusion techniques.

The compounds of the present invention may be administered by any suitable route, preferably in the form of a pharmaceutical composition adapted to such a route, and in a dose effective for the treatment intended. Therapeutically effective doses of the compounds of the present invention required to prevent or arrest the progress of the medical condition are readily ascertained by one of ordinary skill in the art.

Accordingly, the invention provides a class of novel pharmaceutical compositions comprising one or more compounds of the present invention in association with one or more non-toxic, pharmaceutically acceptable carriers and/or diluents and/or adjuvants (collectively referred to herein as "carrier" materials) and if desired other active ingredients. The compounds and composition may for example be administered intravascularly, intraperitoneally, subcutaneously, intramuscularly or topically.

For oral administration, the pharmaceutical composition may be in the form of, for example, a tablet, capsule, suspension or liquid. The pharmaceutical composition is preferably made in the form of a dosage unit contained in a particular amount of the active ingredient. Examples of such dosage units are tablets or capsules. These may with advantage contain an amount of active ingredient from about 1 to 250 mg preferably from about 25 to 150 mg. A suitable daily dose for a mammal may vary widely depending on the condition of the patient and other factors. However, a dose of from about 0.1 to 3000 mg/kg body weight, particularly from about 1 to 100 mg/kg body weight may be appropriate.

The active ingredient may also be administered by injection as a composition wherein, for example, saline, dextrose or water may be used as a suitable carrier. A suitable daily dose is from about 0.1 to 100 mg per kg body weight injected per day in multiple doses depending on the disease being treated. A preferred daily dose would be from about 1 to 30 mg/kg body weight.

The dosage regimen for treating an infectious disease condition with the compounds and/or compositions of this invention is selected in accordance with a variety of factors, including the type, age, weight, sex and medical condition of the patient; the severity of the infection; the route of administration; and the particular compound employed and thus may vary widely.

For therapeutic purposes, the compounds of this invention are ordinarily combined with one or more adjuvants appropriate to the indicated route of administration. If per os, the compounds may be admixed with lactose, sucrose, starch powder, cellulose esters of alkanoic acids, cellulose alkyl esters, talc, stearic acid, magnesium stearate, magnesium oxide, sodium and calcium salts of phosphoric and sulphuric acids, gelatin, acacia, sodium alginate, polyvinylpyrrolidone, and/or polyvinyl alcohol, and thus tableted or encapsulated for convenient administration. Alternatively, the compounds may be dissolved in water, polyethylene glycol, propylene glycol, ethanol, corn oil, cottonseed oil, peanut oil, sesame oil, benzyl

alcohol, sodium chloride, and/or various buffers. Other adjuvants and modes of administration are well and widely known in the pharmaceutical art. Appropriate dosages, in any given instance, of course depend upon the nature and severity of the condition treated, the route of administration, and the species of mammal involved, including its size and any individual idiosyncrasies.

Representative carriers, diluents and adjuvants include for example, water, lactose, gelatin, starches, magnesium stearate, talc, vegetable oils, gums, polyalkylene glycols, petroleum jelly, etc. The pharmaceutical compositions may be made up in a solid form such as granules, powders or suppositories or in a liquid form such as solutions, suspensions or emulsions. The pharmaceutical compositions may be subjected to conventional pharmaceutical operations such as sterilization and/or may contain conventional pharmaceutical adjuvants such as preservatives, stabilizers, wetting agents, emulsifiers, buffers, etc.

Dosage levels of the order from about 1 mg to about 100 mg per kilogram of body weight per day are useful in the treatment of the above-indicated conditions (from about 50 mg to about 5 mg per patient per day). For example, inflammation is effectively treated and anti-pyretic and analgesic activity manifested by the administration from about 25 to about 75 mg of the compound per kilogram of body weight per day (about 75 mg to about 3.75 gram per patient per day). Preferably, from about 5 mg to about 50 mg per kilogram of body weight per daily dosage produces highly effective results (about 250 mg to about 2.5 gm per patient per day).

The amount of active ingredient that may be combined with the carrier materials to produce a single dosage form will vary depending upon the host treated and the particular mode of administration. For example, a formulation intended for the oral administration of humans may contain from 5 mg to 95 mg of active agent compounded with an appropriate and convenient amount of carrier material which may vary from about 5 to 95 percent of the total composition. Dosage unit forms will generally contain between from about 25 mg to about 500 mg of active ingredient.

It will be understood, however, that the specific dose level for any particular patient will depend upon a variety of factors including the activity of the specific compound employed, the age, body weight, general health, sex, diet, time of administration, route of administration, rate of excretion, drug combination and the severity of the particular disease undergoing therapy.

The following Examples are intended to further illustrate the present invention and not to limit the invention in spirit or scope. In the Examples, all parts are parts by weight unless otherwise expressly set forth.

EXAMPLE 1 α-[[4-Methoxyphenyl)methoxy]methyl]-3-pyridineethanol

a) 2,3-Epoxypropyl 4-methoxybenzyl ether

A solution of 4-methoxybenzyl alcohol (800 g, 5.8 mol) in dry tetrahydrofuran (1200 ml) was added dropwise to a stirred slurry of sodium hydride (280 g of a 60% dispersion in mineral oil, 7.0 mol) in dry tetrahydrofuran (600 ml) at -5°C and under a gentle stream of dry nitrogen. The resulting slurry containing a sodium alkoxide was cooled to -5°C. To the cooled slurry was added epibromohydrin (860 g, 6.3 mol) at a rate so as to maintain the temperature of the reaction mixture below 5°C. The reaction mixture was allowed to warm gradually to room temperature and was then stirred for 12 hours. The reaction mixture was filtered and washed with methanol. The filtrate and washings were combined and evaporated to dryness under reduced pressure to yield a crude product (250 g). The crude product was purified by column chromatography (silica gel, chloroform) to yield 2,3-epoxypropyl 4-methoxybenzyl ether as a pale yellow oil having the following physical characteristics:

$^1$H-NMR (δ-$CDCl_3$): 2.70 (m,2H), 3.20 (m,1H), 3.65 (m, 2H), 3.37 (s,3H), 4.73 (s,2H) and 7.15 (q,4H).

b) α-[[(4-Methoxyphenyl)methoxy]methyl]-3-pyridineethanol

A solution of t-butyl lithium (15.7 ml of 1.4M in hexane, 0.022 mol) was added with stirring to dry tetrahydrofuran (100 ml) at -100°C under a nitrogen atmosphere and stirred for 10 minutes. To the resulting yellow solution was added a solution for 3-bromopyridine (3.5 g, 0.022 mol) in dry tetrahydrofuran (5 ml). The resulting green solution was stirred at -100°C for 20 minutes. To the resulting solution was added a cuprous iodide trimethylphosphite complex (6.9 g, 0.022 mol) and the resulting pale brown solution was stirred at -78°C for 5 minutes. To the solution was added a solution of 2,3-epoxypropyl 4-methoxybenzyl ether (4.27 g, 0.022 mol) in dry tetrahydrofuran (10 ml). The reaction mixture was allowed to warm up to

0°C over 2.5 hours and then stirred at room temperature overnight, after which was added a saturated aqueous solution of ammonium chloride (250 ml). The resulting mixture was extracted with ethyl acetate (3x100 ml). The combined extracts were washed with water (4x50 ml), dried over anhydrous magnesium sulphate, filtered, and concentrated under reduced pressure to yield a crude product. The crude product was purified by column chromatography (silica gel, chloroform) and recrystallized from ether-pentane to yield α-[[(4-methoxyphenyl)methoxy]methyl]-3-pyridineethanol as a colorless crystalline solid, having a melting point of 55-56°C and the following physical characteristics:

Elemental analysis: C, 70.20%; H, 7.02%; N, 5.18%; as against calculated values of C, 70.31%; H, 7.01%; N, 5.13% for $C_{16}H_{19}NO_3$.

$^1$H-NMR (δ-$CDCl_3$): 2.40-2.85 (m, 3H), 3.10 (broad s, 1H), 3.30-3.52 (m, 2H), 3.81 (s, 3H), 3.95-4.10 (m, 1H), 4.47 (s, 2H), 6.88 and 7.26 (ABq, 4H), 7.15-7.35 (m, 1H), 7.52-7.58 (m, 1H), 8.40-8.50 (m, 2H); and represented by the structural formula:

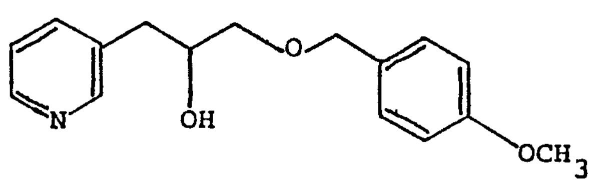

EXAMPLE 2 α-[[(4-Methoxyphenyl)methoxy]methyl]-4-pyridineethanol

Utilizing the procedure described in Example 1(b) but employing 4-bromopyridine in lieu of 3-bromopyridine yielded a crude product. The crude product was purified by column chromatography (silica gel, 10% ethanol in dichloromethane) and recrystallized from diethyl ether to yield α-[[(4-methoxyphenyl)-methoxy]methyl]-4-pyridineethanol as a colorless crystalline solid, having a melting point of 67-68°C and the following physical characteristics:

Elemental analysis: C, 70.07%; H, 7.00%; N, 5.06%; as against calculated values of C, 70.31%; H, 7.01%; N, 5.13%; for $C_{16}H_{19}NO_3$.

$^1$H-NMR (δ-$CDCl_3$): 2.76 (d. 2H), 8.87 (broad s, 1H), 3.30-3.50 (m, 2H), 3.80 (s, 3H), 3.96-4.10 (m, 1H), 4.46 (s, 2H), 6.88 and 7.24 (ABq, 4H), 7.06-7.20 (m, 2H) and 8.25-8.70 (broad s, 2H); and represented by the structural formula:

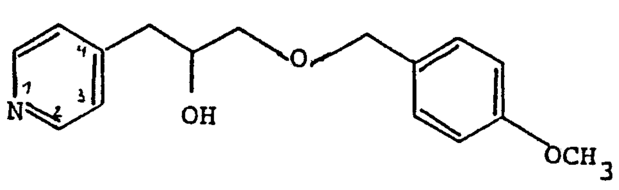

EXAMPLE 3 α-[[(4-Methoxyphenyl)methoxy]methyl]-2-pyridineethanol

Utilizing the procedure described in Example 1(b) but employing 2-brompyridine in lieu of 3-bromopyridine yielded a crude product. The crude product was purified by column chromatography (silica gel, chloroform) to yield α-[[(4-methoxyphenyl)methoxy]methyl]-2-pyridineethanol as a pale-yellow oil, having the following physical characteristics: Elemental analysis: C, 69.81%; H, 7.06%; N, 4.93%; as against calculated values of C, 69.85%; H, 7.03%; N, 5.09% for $C_{16}H_{19}NO_3 \bullet 0.1H_2O$.

$^1$H-NMR (δ-$CDCl_3$): 2.87-3.05 (m, 2H), 3.45-3.57 (m, 2H), 3.79 (s, 3H), 4.16-4.33 (m, 1H), 4.49 (s, 2H), 4.78 (broad s, 1H), 6.86 and 7.26 (ABq, 4H), 7.08-7.20 (m, 2H), 7.55-7.67 (dt, 1H) and 8.45-8.54 (m, 1H); represented by the structural formula:

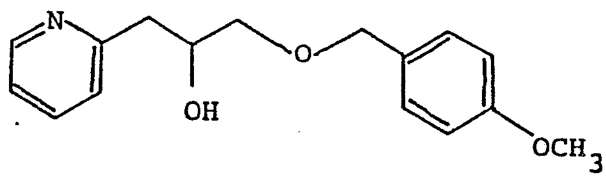

EXAMPLE 4

PAF - Induced Primary Aggregation in Human Platelet-Rich Plasma

Human venous blood was collected from healthy male donors, who had denied any medication during the previous 14 days. Nine volumes of blood were mixed with one volume of 3.24% trisodium citrate. The citrated blood was centrifuged at 160 g for ten minutes at 22°C to obtain platelet rich plasma (PRP). The platelets were then counted on a Coulter counter, and the platelet count was adjusted to 200,000 per µl with plasma. The PRP was then treated with indomethacin dissolved in dimethylsufoxide (10 µg indomethacin per ml PRP). A stock solution of PAF ($C_{16}$ or $C_{18}$ β-acetyl-γ-O-alkyl-L-α-phosphatidylcholine; 1 mg/ml) was prepared as a solution in chloroform-methanol (9:1 v/v). A 10 µl portion of the stock solution was evaporated to dryness under nitrogen and dissolved in 0.15M sodium chloride-0.15M Tris•HCl (90:10 v/v) buffer containing 0.25% bovine albumin. Small volumes of the PAF solution (5-15 µl) were added to 0.5 ml samples of indomethacin-treated PRP and the aggregation trace was recorded using an aggregometer. A dose response curve was obtained and a sub-optimal dose of PAF was determined for use in inhibition studies.

Indomethacin-treated PRP (0.5 ml) was incubated with a test compound dissolved in buffer (ca. pH5) or dimethylsulfoxide for two minutes at 37°C prior to addition of the sub-optimal dose of PAF. The subsequent aggregation trace was recorded and the percent inhibition of PAF primary aggregation was determined by comparison with a control PAF-induced aggregation trace. (Due to variability in PAF-induced aggregation within a given PRP sample, a control PAF-induced aggregation was determined every two or three samples.) An inhibition dose-response curve was determined for each test compound and the $IC_{50}$ value calculated. Table I lists $IC_{50}$'s for illustrative examples of the compounds of this invention.

Table I. PAF-induced primary aggregation in human platelet-rich plasma

| Compound [Example number] | $IC_{50}$ (µM) |
|---|---|
| 1 | 158 |
| 2 | 210 |
| 3 | 750 |

**Claims**

1. A compound of the formula

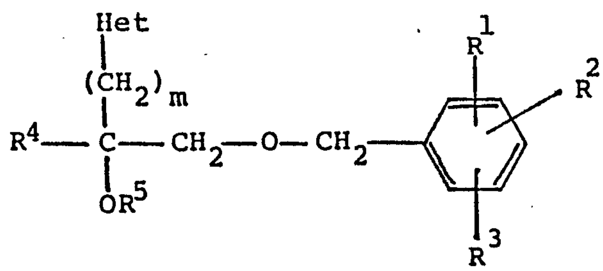

wherein $R^1$, $R^2$ and $R^3$ are independently hydrogen or $C_1$-$C_8$ alkoxy;
$R^4$ is hydrogen or $C_1$-$C_8$ alkyl;
$R^5$ is hydrogen, $-\overset{O}{\overset{\|}{C}}-R^6$, or $C_1$-$C_8$ alkyl,
wherein $R^6$ is $C_1$-$C_8$ alkyl;
m is an integer of from 1 to 4; and Het is pyridiyl;
or a pharmaceutically acceptable acid addition salt thereof.

2. A compound according to Claim 1 wherein m is 1.

3. A compound according to Claim 1 where $R^4$ is hydrogen.

4. A compound according to Claim 1 wherein $R^5$ is hydrogen.

5. A compound according to Claim 1 wherein one of $R^1$, $R^2$ and $R^3$ is $C_1$-$C_8$ alkoxy and the others of $R^1$, $R^2$ and $R^3$ are hydrogen.

6. A compound according to Claim 1 having the formula

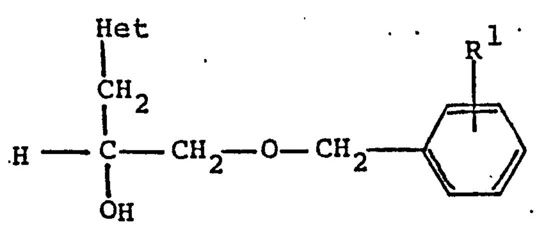

wherein $R^1$ is $C_1$-$C_8$ alkoxy and Het is pyridyl; or a pharmaceutically acceptable acid addition salt thereof.

7. A compound according to Claim 6 having the formula

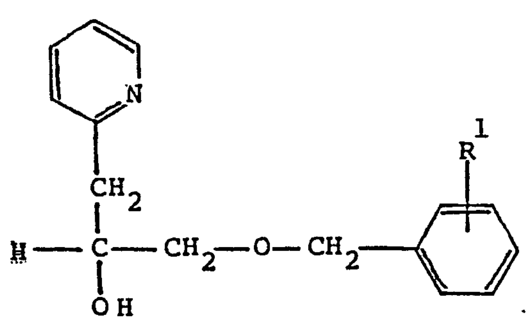

wherein $R^1$ is $C_1$-$C_8$ alkoxy; or a pharmaceutically acceptable acid addition salt thereof.

8. A compound according to Claim 7 which is α[[(4-methoxyphenyl)methoxy]methyl]-2-pyridineethanol.

9. A compound according to Claim 6 having the formula

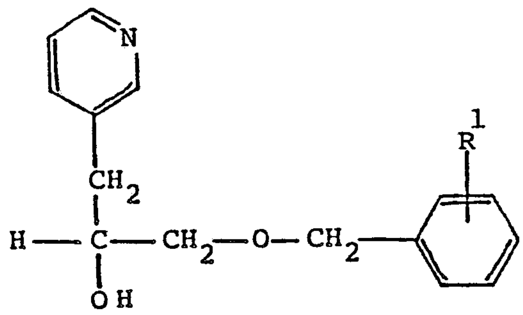

wherein $R^1$ is $C_1$-$C_8$ alkoxy; or a pharmaceutically acceptable acid addition salt thereof.

10. A compound according to Claim 9 which is α[[(4-methoxyphenyl)methoxy]methyl]-3-pyridineethanol.

11. A compound according to Claim 6 having the formula

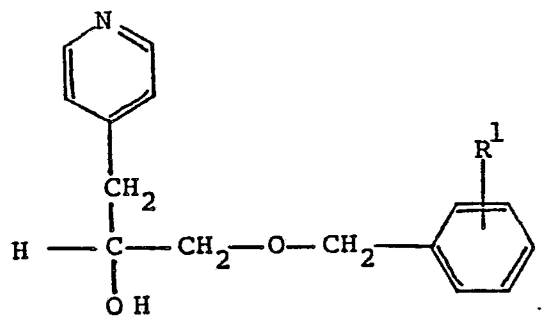

wherein $R^1$ is $C_1$-$C_8$ alkoxy; or a pharmaceutically acceptable acid addition salt thereof.

12. A compound according to Claim 11 which is α[[(4-methoxyphenyl)methoxy]methyl]-4-pyridineethanol.

13. A pharmaceutical composition comprising at least one compound according to Claim 1, together with one or more non-toxic pharmaceutically acceptable carriers.

14. A pharmaceutical composition according to Claim 13 wherein said compound is selected from the group consisting of:
α[[(4-methoxyphenyl)methoxy]methyl]-2-pyridineethanol,
α[[(4-methoxyphenyl)methoxy]methyl]-3-pyridineethanol, and
α[[(4-methoxyphenyl)methoxy]methyl]-4-pyridineethanol.

15. Use of at least one compound of Claims 1 to 12 for the manufacture of a medicament for treating diseases or disorders mediated by platelet-activating factor.

16. Use according to Claim 15 wherein said compound is selected from the group consisting of:
α[[(4-methoxyphenyl)methoxy]methyl]-2-pyridineethanol,
α[[(4-methoxyphenyl)methoxy]methyl]-3-pyridineethanol, and
α[[(4-methoxyphenyl)methoxy]methyl]-4-pyridineethanol.

Claims for the following contracting state: GR

1. A comound of the formula

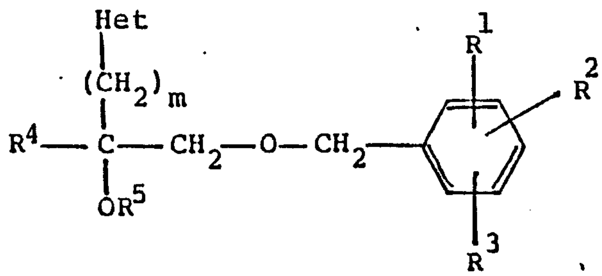

wherein $R^1$, $R^2$ and $R^3$ are independently hydrogen or $C_1$-$C_8$ alkoxy;
$R^4$ is hydrogen or $C_1$-$C_8$ alkyl;
$R^5$ is hydrogen, $-\overset{O}{\overset{\|}{C}}-R^6$, or $C_1$-$C_8$ alkyl,
wherein $R^6$ is $C_1$-$C_8$ alkyl;
m is an integer of from 1 to 4; and Het is pyridyl;
or a pharmaceutically acceptable acid addition salt thereof,

2. A compound according to Claim 1 wherein m is 1.

3. A compound according to Claim 1 where $R^4$ is hydrogen.

4. A compound according to Claim 1 wherein $R^5$ is hydrogen.

5. A compound according to Claim 1 wherein one of $R^1$, $R^2$ and $R^3$ is $C_1$-$C_8$ alkoxy and the others of $R^1$, $R^2$ and $R^3$ are hydrogen.

6. A compound according to Claim 1 having the formula

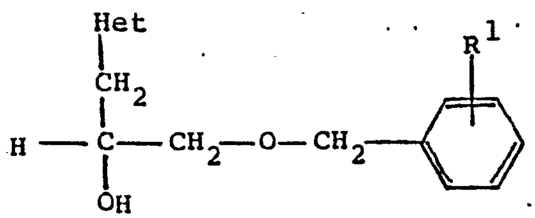

wherein $R^1$ is $C_1$-$C_8$ alkoxy and Het is pyridyl; or a pharmaceutically acceptable acid addition salt thereof.

7. A compound according to Claim 6 having the formula

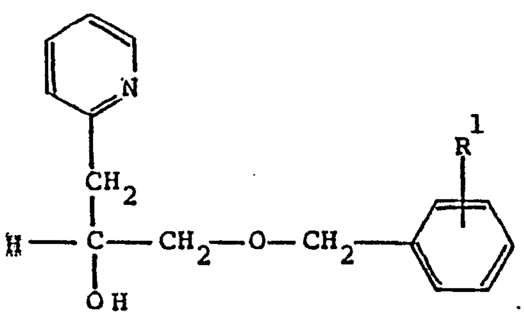

wherein $R^1$ is $C_1$-$C_8$ alkoxy; or a pharmaceutically acceptable acid addition salt thereof.

8. A compound according to Claim 7 which is α[[(4-methoxyphenyl)methoxy]methyl]-2-pyridineethanol.

9. A compound according to Claim 6 having the formula

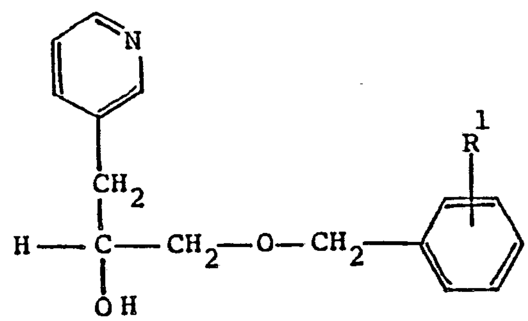

wherein $R^1$ is $C_1$-$C_8$ alkoxy; or a pharmaceutically acceptable acid addition salt thereof.

10. A compound according to Claim 9 which is α[[(4-methoxyphenyl)methoxy]methyl]-3-pyridineethanol.

11. A compound according to Claim 6 having the formula

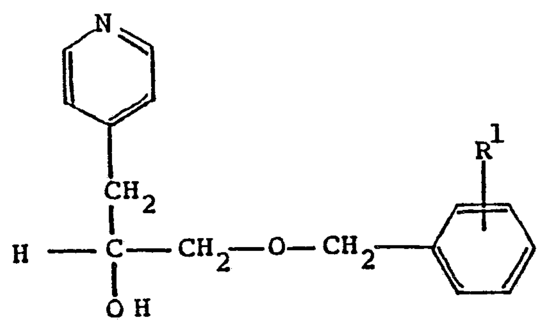

wherein $R^1$ is $C_1$-$C_8$ alkoxy; or a pharmaceutically acceptable acid addition salt thereof.

12. A compound according to Claim 11 which α[[(4-methoxyphenyl)methoxy]methyl]-4-pyridineethanol.

13. Use of at least one compound of Claims 1 to 12 for the manufacture of a medicament for treating diseases or disorders mediated by platelet-activating factor.

14. Use according to Claim 13 wherein said compound is selected from the group consisting of:
α[[(4-methoxyphenyl)methoxy]methyl]-2-pyridineethanol,
α[[(4-methoxyphenyl)methoxy]methyl]-3-pyridineethanol, and
α[[(4-methoxyphenyl)methoxy]methyl]-4-pyridineethanol.

Claims for the following contracting state: ES

1. A process for the production of a-[(phenylmethoxy)methyl]-pyridinealkanol derivatives of the general formula

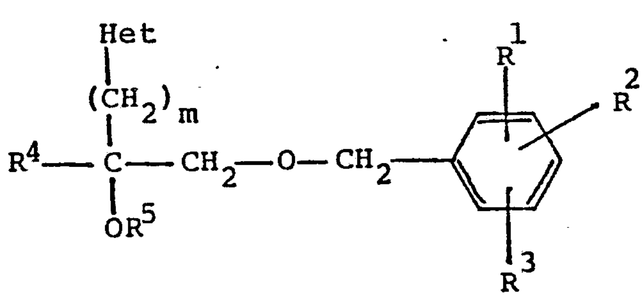

wherein $R^1$, $R^2$ and $R^3$ are independently hydrogen or $C_1$-$C_8$ alkoxy;
$R^4$ is hydrogen or $C_1$-$C_8$ alkyl;
$R^5$ is hydrogen, $-\overset{O}{\overset{\|}{C}}-R^6$, or $C_1$-$C_8$ alkyl,
wherein $R^6$ is $C_1$-$C_8$ alkyl;

m is an integer of from 1 to 4; and Het is pyridyl;
or a pharmaceutically acceptable acid addition salt thereof, characterized in that a benzyl alcohol of the general formula

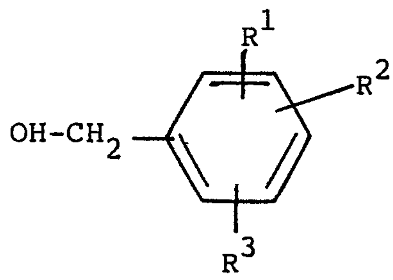

wherein $R^1$, $R^2$ and $R^3$ are defined as above, is reacted with a hydride, the resulting alkoxide is reacted with an epibromhydrin or derivative thereof of the general formula

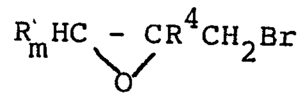

wherein $R^4$ is as defined above and m is an integer of from 1 to 4;
and the resulting epoxyalkyl benzylether derivative of the general formula

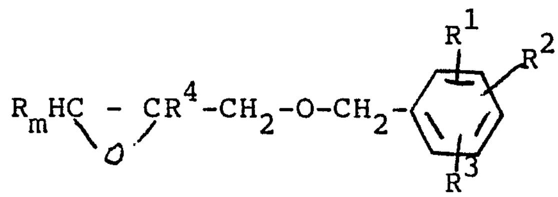

wherein $R^1$, $R^2$, $R^3$ and $R^4$ are as defined above and m is an integer of from 1 to 4;
is reacted with a solution of a bromopyridine of the general formula

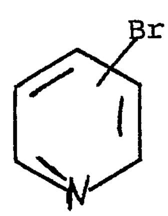

2. A process according to Claim 1 wherein m is 1.
3. A process according to Claim 1 where $R^4$ is hydrogen.
4. A process according to Claim 1 wherein $R^5$ is hydrogen.
5. A process according to Claim 1 wherein one of $R^1$, $R^2$ and $R^3$ is $C_1$-$C_8$ alkoxy and the others of $R^1$, $R^2$ and $R^3$ are hydrogen.
6. A process according to Claim 1 wherein the product obtained has the formula

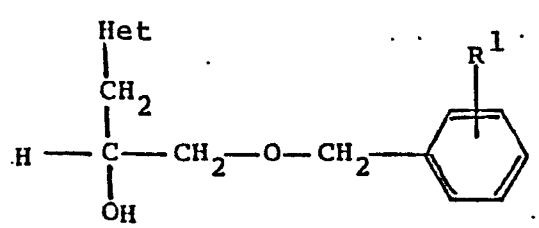

wherein $R^1$ is $C_1$-$C_8$ alkoxy and Het is pyridyl; or a pharmaceutically acceptable acid addition salt thereof.

7. A process according to claim 6 wherein the product obtained has the formula

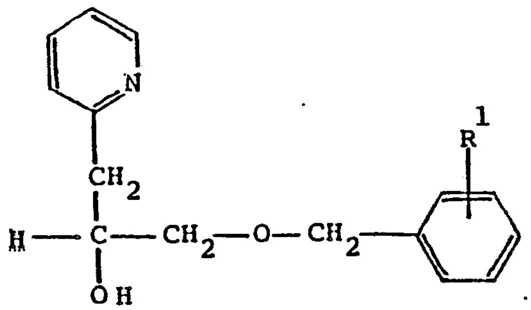

wherein $R^1$ is $C_1$-$C_8$ alkoxy; or a pharmaceutically acceptable acid addition salt thereof.

8. A process according to Claim 7,wherein the product obtained is α[[(4-methoxyphenyl)methoxy]-methyl]-2-pyridineethanol.

9. A process according to Claim 6 wherein the product obtained has the formula

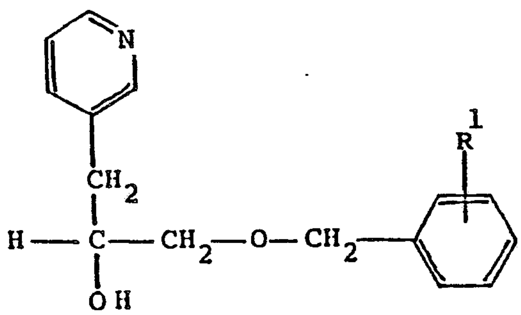

wherein $R^1$ is $C_1$-$C_8$ alkoxy; or a pharmaceutically acceptable acid addition salt thereof.

10. A process according to Claim 9 wherein the product obtained is α[[(4-methoxyphenyl)methoxy]-methyl]-3-pyridineethanol.

11. A process according to Claim 6 wherein the product obtained has the formula

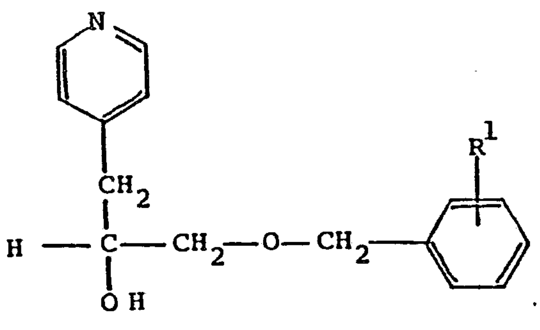

wherein $R^1$ is $C_1$-$C_8$ alkoxy; or a pharmaceutically acceptable acid addition salt thereof.

12. A process according to Claim 11 wherein the product obtained is α[[(4-methoxyphenyl)methoxy]-methyl]-4-pyridineethanol.

13. Use of at least one compound prepared according to Claims 1-12 for the manufacture of a medicament for treating diseases or disorders mediated by platelet-activating factor.

14. Use according to Claim 13 wherein said compound is selected from the group containing of:
α[[(4-methoxyphenyl)methoxy]methyl]-2-pyridineethanol,
α[[(4-methoxyphenyl)methoxy]methyl]-3-pyridineethanol, and
α[[(4-methoxyphenyl)methoxy]methyl]-4-pyridineethanol.

European Patent Office

# EUROPEAN SEARCH REPORT

Application number
EP 87114979.5

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US - A - 4 579 862 (MANLEY)<br>* Column 1, lines 1-65; examples 49,52 *<br>---- | 1-13 | C 07 D 213/30<br>A 61 K 31/44 |
| | | | TECHNICAL FIELDS SEARCHED (Int. Cl.4) |
| | | | C 07 D 213/00 |

The present search report has been drawn up for all claims

| Place of search | Date of completion of the search | Examiner |
|---|---|---|
| VIENNA | 12-01-1988 | HOCHHAUSER |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons

& : member of the same patent family, corresponding document

EPO Form 1503 03 82